# EUROPEAN PATENT APPLICATION

(11) **EP 1 961 738 A1**
(43) Date of publication of application: **27.08.2008**
(21) Application number: 07290196.0
(22) Date of filing: 15.02.2007
(51) Int. Cl.: C07C 281/12, C07D 241/04, C07D 295/135, C07D 295/15, A61K 31/175, A61K 31/4453, A61K 31/5375, A61P 25/28

(54) **New compounds with antiglutamatergic properties and uses thereof**

(71) Applicant: Faust Pharmaceuticals, 67400 Illkirch (FR)
(72) Inventor: Schann, Stephan, 67118 Geispolsheim (FR); Mayer, Stanislas, 67114 Eschau (FR)

(57) **Abstract**

The present invention provides new compounds displaying antiglutamatergic properties. It also provides the use of such compounds for the treatment and/or prophylaxis of conditions and diseases linked to abnormalities in glutamatergic transmission. It further provides a pharmaceutical composition for the treatment and/or prophylaxis of acute and chronic neurodegenerative conditions and disorders.

## Description

The present invention provides new compounds displaying antiglutamatergic properties. It also provides the use of such compounds for the treatment and/or prophylaxis of conditions and diseases linked to abnormalities in glutamatergic transmission. It further provides a pharmaceutical composition for the treatment and/or prophylaxis of acute and chronic neurodegenerative conditions and disorders.

Therapeutic properties of some naphthoquinone derivatives and especially of beta-naphthoquinone derivatives are described in a patent filed in 1960 (BSM924M). This application discloses haemostatic and vitamin properties of such compounds. Other interesting properties such as inhibition of blood platelet aggregation (EP0631777) and acceleration of the proliferation of endothelial cells (EP0631776) with the same beta-naphtoquinone derivatives were displayed in 1993. It also has been shown that various other beta-naphthoquinone derivatives display antitumoral, antimicrobial, antifungal, antiviral and antiparasitic effects.

In 1999, surprising antiglutamatergic properties of known beta naphthoquinone derivatives were described by the French Research team of Israël et al. (EP1196176). They thought that those properties could be useful for the prevention and/or treatment of glutamate cytotoxicity in neurological and psychiatric applications. In fact in the central nervous system (CNS), glutamate is the main excitatory neurotransmitter and is referred to as an excitatory aminoacid, and gamma-aminobutyric acid (GABA) is the main inhibitory neurotransmitter. The balance between excitation and inhibition is of utmost importance to CNS functions, and dysfunctions of either of the two can be related to various neurological disorders.

Glutamate binds or interacts with one or more glutamate receptors, which can be differentiated pharmacologically into different classes and subtypes. Glutamate is ubiquitously distributed in the nervous system in high concentrations, especially in the brain and spinal cord of mammals, where it is working at a variety of excitatory synapses being thereby involved in virtually all physiological functions such as motor control, vision, central control of heart, processes of learning and memory. However, a large number of studies have established that cellular communication involving glutamate can also lead to a mechanism of neuronal cell damage or loss. This mechanism is known as excitotoxicity. Excitotoxicity has a role in acute disorders (traumatic, anoxic, hypoglycaemic or vascular disorders), as well as in chronic neurodegenerative disorders.

Glutamatergic transmission has been shown to be clearly involved in the pathophysiology of a number of neuronal damages and injuries. Many nervous system disorders including epilepsy and chronic or acute degenerative processes such as for example Alzheimer's disease, Huntington's disease, Parkinson's disease and amyotrophic lateral sclerosis (Mattson et al., Neuromolecular Med., 65, 2003), but also AIDS-induced dementia, multiple sclerosis, spinal muscular atrophy, retinopathy, stroke, ischemia, hypoxia, hypoglycaemia and various traumatic brain injuries, involve neuronal cell death caused by imbalanced levels of glutamate. It has also been shown that drug-induced neurotoxicity, for example neurotoxic effects of methamphetamine (METH) on striatal dopaminergic neurons, could actually be mediated by over-stimulation of the glutamate receptors (Stephans and Yamamoto, 1994, Synapse, 17, 203-209). Antidepressant and anxiolytic-like effects of compounds acting on glutamate have also been observed on mice, suggesting that glutamatergic transmission is implicated in the pathophysiology of affective disorders such as major depression and anxiety (Cryan et al., 2003, Eur. J. Neurosc., 17(11):2409-17). Consequently, any compound able to modulate glutamatergic signalling or function would constitute a promising therapeutic compound for many disorders of the nervous system.

Among abnormalities in glutamatergic transmission, excitotoxicity contributes to neurodegenerative disorders and diseases. Therefore, antiglutamatergic strategies are currently developed especially to fight characteristic symptoms of Amyotrophic Lateral Sclerosis, Parkinson's disease and Alzheimer's disease. Drugs able to modulate the transmission of glutamate in a patient in need thereof are referred to as antiglutamatergic or neuroprotective compounds. Riluzole (Rilutek®, Aventis Pharma Inc.), topiramate (Epitomax®, Janssen Cilag) and amantadine (Mantadix®, Bristol-Myers Squibb) are such antiglutamatergic compounds able to modulate, directly or indirectly, glutamate levels. Those compounds seem efficient in the treatment and/or prevention of symptoms linked to various diseases among which epilepsy, Parkinson's disease and Amyotrophic Lateral Sclerosis, but also migraine, depression and bipolar disorders.

In consideration of most of the available antiglutamatergic compounds, which serve as research tools and also as potential therapeutic compounds, we must admit that most of them display a lack of potency and/or selectivity. Accordingly, there is still a need for safe and efficacious antiglutamatergic compounds.

It is an object of the present invention to provide new beta-naphtoquinone derivatives with high solubility properties, and displaying antiglutamatergic properties. This and other objects and advantages of the invention will be described and detailed in the following description.

The present invention provides compounds of formula (I): 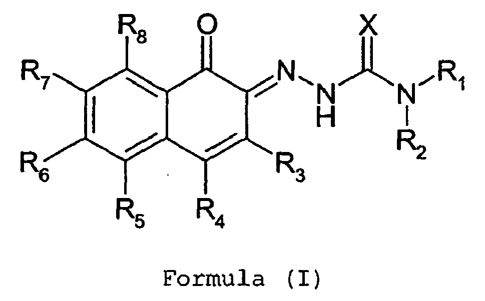 wherein:
**X** is S or O;
**R₁** and **R₂** are independently from each other hydrogen, alkyl, or can be linked to generate heterocycloalkyl;
**R₃, R₅, R₆, R₇** and **R₈** are independently from each other hydrogen, halogen, CN, CF₃, OCF₃, alkyl, COOH, CONH(alkyl), CON(alkyl)(alkyl), CO(heterocycloalkyl), OH, O-(alkyl), S-(alkyl), NH₂, NH (alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl;
**R₄** is hydrogen, halogen, CN, CF₃, OCF₃, alkyl, COOH, CONH(alkyl), CON(alkyl)(alkyl), CO(heterocycloalkyl), O-(alkyl), S-(alkyl), NH₂, NH(alkyl), N-(alkyl)(alkyl), NHCO(alkyl), N(Alkyl)CO(alkyl), NHSO₂(alkyl), N (Alkyl) SO₂(alkyl), heterocycloalkyl, aryl or heteroaryl;
With the proviso that **R₁, R₂, R₃, R₄, R₅, R₆, R₇** and **R₈** be not hydrogen all together;
The two Adjacent R groups **R₃-R₄, R₄-R₅, R₅-R₆, R₆-R₇** and **R₇-R₈** can be linked to generate a 5 to 8 member ring with atoms selected from C, N, O, S and P;
as well as pharmaceutically acceptable salts thereof, a prodrug of the compound or the salt, or a solvate or hydrate of the compound, the salt or the prodrug.

As used herein, the term **"alkyl"** includes straight or branched chain saturated hydrocarbon residues with 1-8 carbon atoms, such as methyl, ethyl, n-propyl, isopropyl, n-butyl, i-butyl, s-butyl and t-butyl. These alkyls can be further substituted with groups such as COOH, CONH(lower alkyl), CON(lower alkyl)(lower alkyl), CO(heterocycloalkyl), heterocycloalkyl, CF₃, OH, O-(lower alkyl), NH2, NH-(lower alkyl), N-(lower alkyl)(lower alkyl), aryl or heteroaryl.
As used herein, the term **"lower alkyl"** includes straight or branched chain saturated hydrocarbon residues with 1-4 carbon atoms, such as methyl, ethyl, n-propyl, isopropyl, n-butyl, i-butyl, s-butyl or t-butyl.
The term **"aryl"** refers to a 6-10 atoms aromatic hydrocarbon ring or fused aromatic hydrocarbon ring system containing at least one unsaturated aromatic ring. Examples of the term "aryl" are phenyl, naphthyl and 1,2,3,4-tetrahydronaphthyl. These aryls can be further substituted with groups such as halogen, CN, CF₃, OCF₃, lower alkyl, COOH, CONH(lower alkyl), CON(lower alkyl)(lower alkyl), CO(heterocycloalkyl), OH, O-(alkyl), S-(alkyl), NH₂, NH-(lower alkyl) N-(lower alkyl)(lower alkyl) or heterocycloalkyl.
The term **"heteroaryl"** means a 5-10 atoms aromatic ring or fused aromatic rings containing one or more O, S, or N atoms. Examples of heteroaryls include pyridinyl, quinolinyl, With the proviso that **R₃, R₄, R₅, R₆, R₇** and **R₈** be not hydrogen all together;
The two Adjacent R groups **R₃-R₄, R₄-R₅, R₅-R₆, R₆-R₇** and **R₇-R₈** can be linked to generate a 5 to 8 member ring with atoms selected from C, N, O, S and P;
as well as pharmaceutically acceptable salts thereof, a prodrug of the compound or the salt, or a solvate or hydrate of the compound, the salt or the prodrug.

In a particular embodiment, the invention concerns a compound of formula (II), wherein:
**X** is S or 0;
**R₃, R₅, R₆, R₇** and **R₈** is hydrogen,
**R₄** is halogen, CN, CF₃, OCF₃, alkyl, COOH, CONH (alkyl), CON(alkyl)(alkyl), CO(heterocycloalkyl), O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

In another particular embodiment, the invention concerns a compound of Formula (III), wherein:
**R₃, R₅, R₆, R₇** and **R₈** is hydrogen;
**R₄** is halogen, CN, CF₃, OCF₃, alkyl, COOH, CONH(alkyl), CON(alkyl)(alkyl), CO(heterocycloalkyl), O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

In a further embodiment, the invention concerns a compound of formula (II), wherein:
**X** is S or O;
**R₃, R₄, R₅, R₇** and **R₈** is hydrogen,
**R₆** is halogen, CN, CF₃, OCF₃, alkyl, COOH, CONH (alkyl), CON(alkyl)(alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

In yet a further embodiment, the invention concerns a compound of Formula (III), wherein:
**R₃, R₄, R₅, R₇** and **R₈** is hydrogen;
**R₆** is halogen, CN, CF₃, OCF₃, alkyl, COOH, CONH (alkyl), CON(alkyl)(alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

In a preferred embodiment, the invention concerns a compound of formula (II), wherein:
**X** is S or O;
**R₃, R₄, R₅, R₆** and **R₈** is hydrogen,
**R₇** is halogen, CN, CF₃, OCF₃, alkyl, COOH, CONH(alkyl), CON(alkyl)(alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

In yet another preferred embodiment, the invention concerns a compound of Formula (III), wherein:
**R₃, R₄, R₅, R₆** and **R₈** is hydrogen;
**R₇** is halogen, CN, CF₃, OCF₃, alkyl, COOH, CONH(alkyl), CON(alkyl) (alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

In a specific embodiment, the invention concerns a compound of formula (II), wherein:
**X** is S or O;
**R₃, R₄, R₅, R₆** and **R₇** is hydrogen,
**R₈** is halogen, CN, CF₃, OCF₃, alkyl, COOH, CONH(alkyl), CON (alkyl) (alkyl), CO(heterocycloalkyl), OH, O-(alkyl), S-(alkyl), NH₂, NH(alkyl), N- (alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

In yet another specific embodiment, the invention concerns a compound of Formula (III), wherein:
**R₃, R₄, R₅, R₆** and **R₇** is hydrogen;
**R₈** is halogen, CN, CF₃, OCF₃, alkyl, COOH, CONH(alkyl), CON(alkyl)(alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl,
as well as pharmaceutically acceptable salt thereof, a prodrug of the compound or the salt, or a solvate or hydrate of the compound, the salt or the prodrug.

Preferred compounds of formula (I), (II) or (III) according to the invention are:
- 4-methylnaphthalene-1,2-dione 2-semicarbazone
- 4-bromonaphthalene-1,2-dione 2-semicarbazone
- 4-(hydroxymethy)naphthalene-1,2-dione 2-semicarbazone
- 4-(methylhydroxymethy)naphthalene-1,2-dione 2-semicarbazone
- 4-(morpholin-4-ylmethyl)naphthalene-1,2-dione2-semicarbazone
- 4-(ethylamine-4-ylmethyl)naphthalene-1,2-dione2-semicarbazone
- 6-methoxynaphthalene-1,2-dione 2-semicarbazone
- *6-morpholin-4-ylnaphthalene-1,2-dione 2-semicarbazone*
- 6-bromonaphthalene-1,2-dione 2-semicarbazone
- 7-(2-morpholin-4-yl-ethoxy) naphtalene-1,2-dione2-semicarbazone
- 7-(2-Dimethylamino-ethoxy)naphtalene-1,2-dione-2-semicarbazone
- N-{7-[(aminocarbonyl)hydrazono]-8-oxo-7,8-dihydronaphthalen-1-yl}-3-piperidin-1-ylpropanamide
- tert-butyl-7-[(aminocarbonyl)hydrazono]-8-oxo-7,8-dihydronaphthalen-1-ylcarbamate
- 7-bromonaphthalene-1,2-dione 2-semicarbazone
8-aminonaphthalene-1,2-dione 2-semicarbazone

The compounds of general formula (I), (II) or (III) and their pharmaceutically acceptable salts can be manufactured according to methods described in the following schemes:

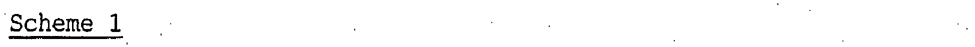

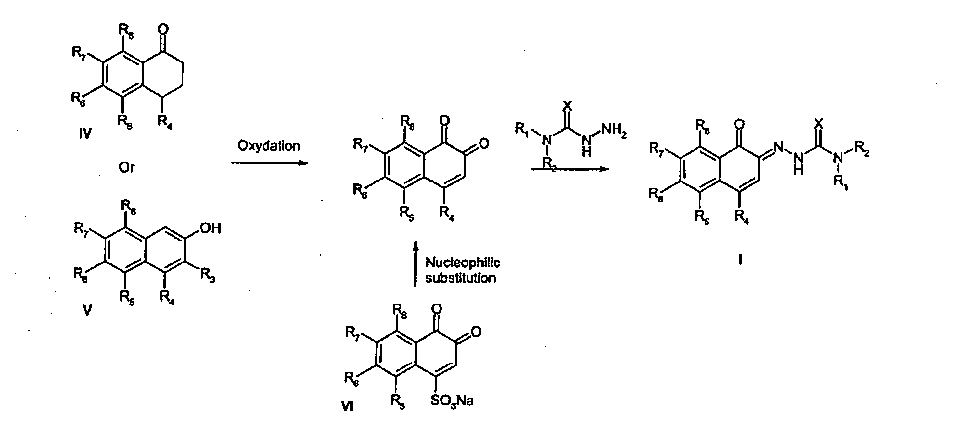

Precursor IV, V and VI could be made according to the state of the art procedure.

### Compounds I could be further functionalized as outlined in scheme 2

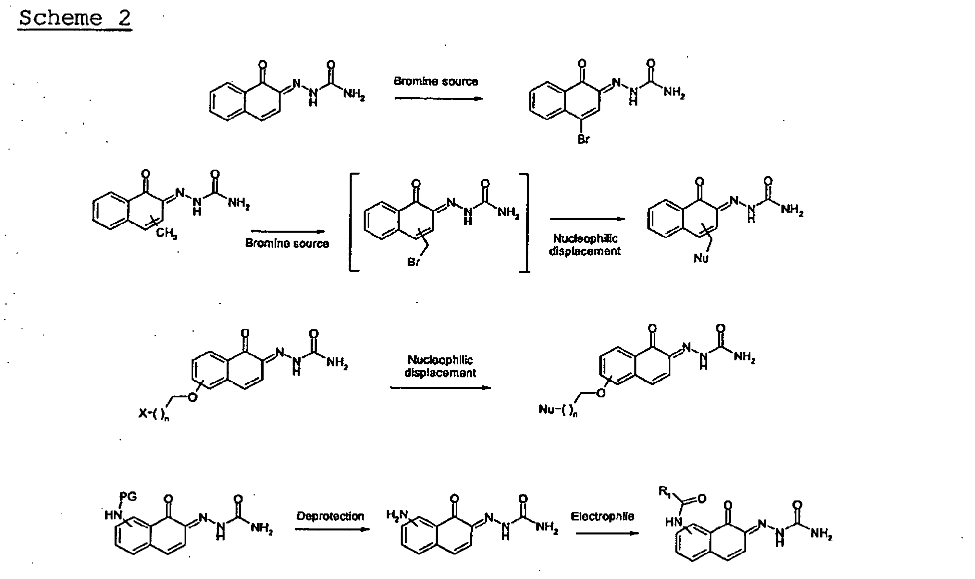

The invention further provides the use of a compound of formula (I), (II) or (III) in the manufacture of a drug for use in the treatment and/or prophylaxis of conditions and diseases linked to abnormalities in glutamatergic transmission.

The conditions and diseases linked to abnormalities in glutamatergic transmission are epilepsy, including newborn, infantile, childhood and adult syndromes, partial (localization-related) and generalized epilepsies, with partial and generalized, convulsive and non-convulsive seizures, with and without impairment of consciousness, and status epilepticus; Dementias, including dementias of the Alzheimer's type (DAT), Pick's disease, vascular dementias, Lewy-body disease, dementias due to metabolic, toxic and deficiency diseases (including alcoholism, hypothyroidism, and vitamin B12 deficiency), AIDS-dementia complex, Creutzfeld-Jacob disease and atypical subacute spongiform encephalopathy; Parkinsonism and movement disorders, including Parkinson's disease, multiple system atrophy, progressive supranuclear palsy, hepatolenticular degeneration, chorea (including Huntington's disease and hemiballismus), athetosis, dystonias (including spasmodic torticollis, occupational movement disorder, Gilles de la Tourette syndrome), levo-dopa induced dyskinesias, tardive dyskinesias and other drug induced dyskinesias and movement disorders, tremor and myoclonus; Motor neuron disease or amyotrophic lateral sclerosis (ALS); Other neurodegenerative and/or hereditary disorders of the nervous system, including spinocerebrellar degenerations such as Friedrich's ataxia and other hereditary cerebellar ataxias, predominantly spinal muscular atrophies, hereditary neuropathies, and phakomatoses; Disorders of the peripheral nervous system, including trigeminal neuralgia, facial nerve disorders, disorders of the other cranial nerves, nerve root and plexus disorders, mononeuritis such as carpal tunnel syndrome and sciatica, hereditary and idiopathic peripheral neuropathies, inflammatory and toxic neuropathies; Deafness, tinnitus, vertigo. Multiple sclerosis and other demyelinating diseases of the nervous system; Infantile cerebral palsy (spastic), monoplegic, paraplegic or tetraplegic; Hemiplegia and hemiparesis, flaccid or spastic, and other paralytic syndromes; Cerebrovascular disorders, including subarachnoid hemorrhage, intracerebral hemorrhage, occlusion and stenosis of precerebral arteries, occlusion of cerebral arteries including thrombosis and embolism, brain ischemia, stroke, transient ischemic attacks, atherosclerosis, cerebrovascular dementias, aneurysms, cerebral deficits due to cardiac bypass surgery and grafting; Migraine, including classical migraine and variants such as cluster headache; Headache; Myoneural disorders including myasthenia gravis, acute muscle spasms, myopathies including muscular dystrophies, myotonias and familial periodic paralysis; Disorders of the eye and visual pathways , including retinal disorders, and visual disturbances; Intracranial trauma/injury and their sequels; Trauma/injury to nerves and spinal cord and their sequels; Poisoning and toxic effects of non medicinal substances; Accidental poisoning by drugs, medicinal substances and biologicals acting on the central, peripheral and autonomic system; Neurological and psychiatric adverse effects of drugs, medicinal and biological substances; Disturbance of sphincter control and sexual function; Mental disorders usually diagnosed in infancy, childhood or adolescence, including : mental retardation, learning disorders, motor skill disorders, communication disorders, pervasive developmental disorders, attention deficit and disruptive behaviour disorders, feeding and eating disorders, TIC disorders, elimination disorders; Delirium and other cognitive disorders; Substance related disorders including: alcohol-related disorders, nicotine-related disorders, disorders related to cocaine, opioids, cannabis, hallucinogens and other drugs; Schizophrenia and other psychotic disorders; Mood disorders, including depressive disorders and bipolar disorders; Anxiety disorders, including panic disorders, phobias, obsessive-compulsive disorders, stress disorders, generalized anxiety disorders; Eating disorders, including anorexia and bulimia; Sleep disorders, including dyssomnias (insomnia, hypersomnia, narcolepsy, breathing related sleep disorder) and parasomnias; Restless legs syndrome; Endocrine and metabolic diseases including diabetes, disorders of the endocrine glands, hypoglycaemia; Acute and chronic pain; Nausea and vomiting; Irritable bowel syndrome.

The present invention further provides a pharmaceutical composition comprising a pharmaceutically effective amount of a compound of the formula (I), (II) or (III), or pharmaceutically acceptable salts thereof, in combination with a pharmaceutically acceptable carrier, diluent or excipient.

According to the invention, the terms "treatment and/or prophylaxis" refer to a process that is intended to produce a beneficial change in the condition of a mammal, e.g., a human, often referred to as a patient. A beneficial change can, for example, include one or more of: restoration of function, reduction of symptoms, limitation or retardation of progression of a disease, disorder, or condition or prevention, limitation or retardation of deterioration of a patient's condition, disease or disorder, improvement of the patient's quality of life.

In another embodiment, the invention provides a pharmaceutical composition for the treatment and/or prophylaxis of a disease or condition selected from epilepsy, dementias (including dementias of the Alzheimer's type, vascular dementias, AIDS-dementia complex, etc...), parkinsonism and movement disorders (including Parkinson's disease, Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye and visual pathways, intracranial trauma/injury, trauma/injury to nerves and spinal cord, poisoning and toxic effects of non medicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, restless legs syndrome, endocrine and metabolic diseases (diabetes, hypoglycaemia), acute and chronic pain; nausea and vomiting; irritable bowel syndrome.

The compounds of the invention may be administered in a form suitable for oral use, for example a tablet, capsule, aqueous or oily solution, suspension or emulsion; for topical use including transmucosal and transdermal use, for example a cream, ointment, gel, aqueous solution or suspension, salve, patch or plaster; for nasal use for example a snuff, nasal spray or nasal drops; for vaginal or rectal use, for example a suppository; for administration by inhalation, for example a finely divided powder or a liquid aerosol; for sub-lingual or buccal use, for example a tablet or a capsule; or for parenteral use (including intravenous, subcutaneous, intramuscular, intravascular or infusion), for example a sterile aqueous solution or suspension, or depot injection formulation. In general, the above compositions may be prepared in a conventional manner using well known excipients, using standard techniques, including controlled release technologies, such as gelatin, lipid, gel depot, liposome and/or microcapsule based systems well known to those skilled in the art of pharmacy.

For an oral administration, the compounds of the invention will generally be provided in the form of tablets or capsule or as an aqueous solution or suspension.

Tablets for oral use may include the active ingredient mixed with pharmaceutically acceptable excipients such as inert diluents (such as sodium and calcium carbonate, sodium and calcium phosphate, or lactose), disintegrating agents (such as corn starch or alginic acid), binding agents (starch or gelatin), lubricating agents (magnesium stearate, stearic acid or talc), sweetening agents, flavoring agents, coloring agents and preservatives. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or oil such as peanut oil, liquid paraffin or olive oil.

For intramuscular, intraperitoneal, subcutaneous and intravenous use, the compounds of the invention will generally be provided in sterile aqueous solutions (such as Ringer's solution or isotonic sodium chloride) or suspensions (such as cellulose derivatives, sodium alginate, polyvinylpyrrolidone and gum tragacanth with a wetting agent such as lecithin, and a preservative such as ethyl and n-propyl p-hydroxybenzoate), buffered to an appropriate pH and isotonicity.

Transdermal formulations include membrane permeation systems, multi-laminate adhesive dispersion systems and matrix dispersion systems. Transdermal delivery also includes the use of electrically aided transport and skin penetration enhancers.

The preferred route of administration is the *per os* or oral administration.

It will be appreciated that the dosage levels used may vary over quite a wide range depending upon the compound used, the severity of the condition exhibited by the patient and the patient's body weight. However, without commitment to a rigid definition of dosages, it may be stated that a daily dosage of the active ingredient is 100 µg to 800 mg.

Other characteristics and advantages of the invention are given in the following examples in which reference is made to:

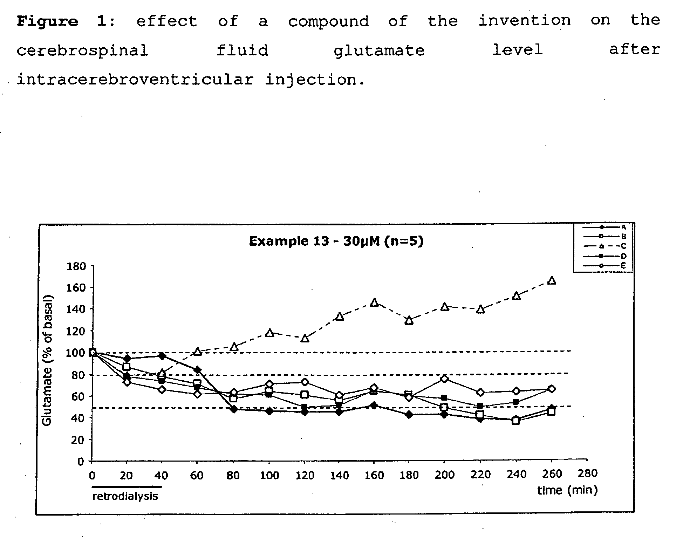

- Figure 1 which represents the effect of a compound of the invention on the cerebrospinal fluid glutamate level after intracerebroventricular (i.c.v.) injection.

It will be appreciated that the invention is described by way of example only and modification of detail may be made without departing from the scope of the invention.

### Experimental:

### General procedure I: formation of 4 substituted 1,2-naphtoquinone from sodium 1,2-naphtoquinone-4-sulphonate.

To a suspension of sodium 1,2-naphtoquinone-4-sulphonate (260 mg, 1.0 mmol) in a 1 N aqueous solution of K₂CO₃ (2 mL), nucleophile (1.1 mmol, 1.1 equiv.) was added. The reaction mixture was stirred for 5 Hrs. A precipitate was filtered off, washed with water (3x5 mL), CH₃CN (1 mL) then Et₂O (3x2 mL). Solid was dried under reduced pressure at 50°C.

### Compound 1: 4-morpholin-4-ylnaphthalene-1,2-dione

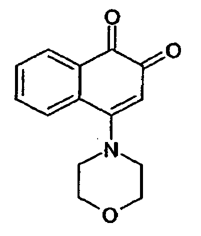

Compound 1 was obtained according to the general procedure I using morpholine as nucleophile, as a red solid (104 mg, 42 %). The reaction was completed within 3.0 Hrs.
¹H-NMR (400 MHz, DMSO): 3.37 (m, 4H, (CH₂)₂-N), 3.81 (m, 4H, (CH₂)₂-O), 5.93 (s, 1H, Ar), 7.63 (t, *j* 7.5 Hz, 1H, Ar), 7.69 (dd, *j* 7.8 Hz, *j* 1.0 Hz, 1H, Ar), 7.77 (bs, 1H, Ar), 7.94 (d, *J* 7.5 Hz, 1H, Ar).
M/Z (M+H)⁺ = 244.

### Compound 2: 4-(4-methylpiperazin-1-yl)naphthalene-1,2-dione

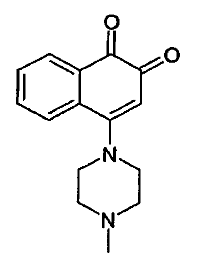

Compound 2 was obtained according to the general procedure I using N-methylpiperazine as nucleophile, as a bright red solid (291 mg, 57 %). The reaction was completed within 1.5 Hrs.
¹H-NMR (400 MHz, DMSO): 2.26 (s, 3H, CH₃N), 2.54 (m, 4H, (CH₂)₂-N), 3.37 (m, 4H, (CH₂)₂-N), 5.93 (m, 1H, Ar), 7.63-7.93 (m, 4H, Ar).
M/Z (M+H)⁺ = 257.

### General procedure II: formation of substituted 1,2-naphtoquinone from tetralone derivatives.

Under inert atmosphere, to a solution of tetralone derivatives (1 mmol) in glacial acetic acid (2 mL), selenium dioxide (250 mg, 2.2 equiv.) was added. The reaction mixture was warmed 15 Hrs at 60°C. The reaction mixture was then cooled to room temperature and was concentrated to dryness.
purification (eluent CycHex:EtOAc 70:30 - Rf: 0.3). The orange residue was taken in Et₂O (5 mL), the resulting solid was filtered off, washed with Et₂O (2x5 mL) and dried under reduced pressure at 50°C for 15 Hrs to give a yellowish solid (271 mg, 52 %).
¹H-NMR (400 MHz, DMSO): 2.37 (s, 3H, CH₃), 6.41 (d, *j* 1.3 Hz, 1H, Ar), 7.61 (td, *j* 7.6 Hz, *j* 1.1 Hz, 1H, Ar), 7.68 (d, *j* 7.6 Hz, 1H, Ar), 7.79 (td, *j* 7.6 Hz, *j* 1.3, 1H, Ar), 7.97 (dd, *j* 7.6 Hz, *j* 1.1 Hz, 1H, Ar).
M/Z (M+H)⁺ = 173.

### Compound 5: 7-bromonaphthalene-1,2-dione

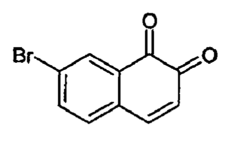

Compound 5 was obtained according to the general procedure II from 7-bromo-α-tetralone, followed by chromatography purification (eluent CycHex:EtOAc 70:30 - Rf: 0.3). The deep brown residue was taken in Et₂O (10 mL), the resulting solid was filtered off, washed with Et₂O (5x2 mL) and dried under reduced pressure at 50°C for 15 Hrs to give a brown solid (284 mg, 47 %).
¹H-NMR (400 MHz, DMSO): 6.43 (d, *j* 10.2 Hz, 1H, Ar), 7.55 (d, *j* 8.1 Hz, 1H, Ar), 7.64 (d, *j* 10.2 Hz, 1H, Ar), 7.95 (dd, *j* 8.1 Hz, *j* 2.0, 1H, Ar), 7.99 (d, *j* 2.0 Hz, 1H, Ar).

### Compound 3: 6-methoxynaphthalene-1,2-dione

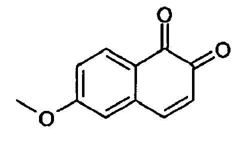

Compound 3 was obtained according to the general procedure II from 6-methoxy-α-tetralone, followed by chromatography purification (eluent CycHex:EtOAc 50:50 - Rf: 0.3). The residue was taken in Et₂O, the resulting solid was filtered off, washed with Et₂O and dried under reduced pressure at 50°C for 15 Hrs to give a brown solid (81 mg, 43 %).
¹H-NMR (400 MHz, DMSO): 3.90 (s, 3H, CH₃-O), 6.40 (d, *j* 10.1 Hz, 1H, Ar), 7.08 (dd, *j* 8.6 Hz, j 2.6 Hz, 1H, Ar), 7.19 (d, *j* 2.6 Hz, 1H, Ar), 7.61 (d, *j* 10.1 Hz, 1H, Ar), 7.93 (d, *j* 8.6 Hz, 1H, Ar).
M/Z (M+H)⁺ = 189.

### Compound 4: 4-methylnaphthalene-1,2-dione

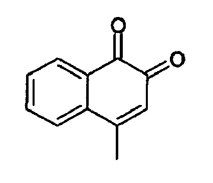

Compound 4 was obtained according to the general procedure II from 4-methyl-1-tetralone, followed by chromatography M/Z (M+H)⁺ = 237.

### General procedure III: formation of substituted 1,2-naphtoquinone from 2-naphtol derivatives.

Under inert atmosphere, to a solution of 2-naphthol derivative (0.5 mmol) in anhydrous THF (10 mL), benzenselenic anhydride (500 mg, 2.0 equiv.) was added. The reaction mixture was warmed 15 min at 60°C. The reaction mixture was then cooled to room temperature, hydrolyzed (20 mL) and then extracted with EtOAc (40 mL). The organic layer was washed with a saturated NaHCO₃ solution (30 mL), a saturated NaCl solution (25 mL) dried over MgSO₄, then concentrated to dryness. The residue was taken in Et₂O (5 mL); the resulting precipitate was collected, washed with Et₂O (2x2 mL), pentane (3x2 mL) then was dried under reduced pressure at 50°C for 15 Hrs.

### General procedure IV: formation of substituted 1,2-naphtoquinone from 2-naphtol derivatives (alternative work-up).

Under inert atmosphere, to a solution of 2-naphthol derivative (0.5 mmol) in THF (10 mL), benzenselenic anhydride (500 mg 2.0 equiv.) was added. The reaction mixture was warmed 15 min at 60°C. The reaction mixture was then cooled to room temperature, hydrolyzed (50 mL) and then cyclohexane was added (60 mL). The biphasic system was well shaken. Insoluble material was filtered off, washed with water (5 mL), cyclohexane (2x3 mL) and pentane (2x2 mL). The solid was dried under reduced pressure at 50°C for 15 Hrs.

### Compound 6: 6-bromonaphthalene-1,2-dione.

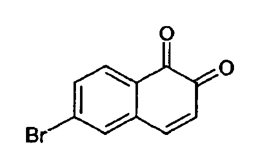

Compound 6 was obtained according to the general procedures III and IV from 6-bromo-2-naphthol, as a red solid (50 mg, 42 % and 60 mg 50 % respectively).
¹H-NMR (400 MHz, DMSO): 6.46 (d, *j* 9.7 Hz, 1H, Ar), 7.50-7.90 (bm, 4H, Ar).
M/Z (M+H)⁺ = 237.

### General procedure V: Amination of bromo-2-naphtol by Buchwald-Hartwig reaction.

Under inert atmosphere, to a solution of bromo-2-naphtol derivative (2.0 mmol) and palladium acetate (100 mg, 0.2 equiv.) in toluene (10 mL), amine (1.4 equiv.), 2,8,9-triisopropyl-2,5,8,9-tetraaza-1-phosphabicyclo(3.3.3)undecane (250 mg 0.4 equiv.) and a 1 N solution of LiHMDS in THF (6 mL - 3.0 equiv.) were successively added. The reaction mixture was warmed 14 Hrs at 80°C, then hydrolyzed at room temperature by a mixture of saturated ammonium chloride solution and ice (100 mL 1:1). A precipitate was obtained. Solid was filtered off, washed with water (2x5 mL), toluene:water (10 mL 1:1) and then was dried under reduced pressure at 50°C for 15 Hrs.

### Compound 7: 6-morpholin-4-yl-2-naphthol

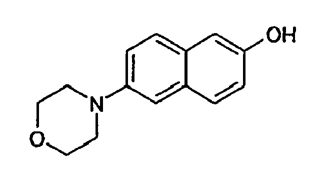

Compound 7 was obtained according to the general procedure V from 6-bromo-2-naphthol and morpholine, as a brown solid in 58 % yield. Compound 7 was used in the next step without further purification.
¹H-NMR (400 MHz, DMSO): 3.12 (m, 4H, (CH₂)₂-N), 3.77 (m, 4H, (CH₂)₂-O), 6.98-7.00 (m, 2H, Ar), 7.08 (d, *j* 2.2 Hz, 1H, Ar), 7.26 (dd, *j* 8.9 Hz, *j* 2.4 Hz, 1H, Ar), 7.57 (m, 2H, Ar), 9.39 (bs, 1H, OH).
M/Z (M+H)⁺ = 230.

### Compound 8: 6-(4-methylpiperazin-1-yl)-2-naphthol

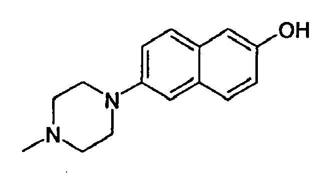

Compound 8 was obtained according to the general procedure V from 6-bromo-2-naphthol and methylpiperazine, as a brown solid in 73 % yield. Compound 8 was used in the next step without further purification.
¹H-NMR (400 MHz, DMSO): 2.25 (s, 3H, CH₃N), 3.15 (m, 4H, (CH₂)₂-N), 3.35 (under HOD signal) (m, 4H, (CH₂)₂-N), 6.98 (m, 2H, Ar), 7.07 (d, *j* 2.4 Hz, 1H, Ar), 7.25 (dd, *j* 9.0 Hz, *j* 2.4 Hz, 1H, Ar), 7.53 (d, *j* 9.0 Hz, 1H, Ar), 7.57 (d, *j* 8.5 Hz, 1H, Ar), 9.37 (bs, 1H, OH).
M/Z (M+H)⁺ = 243.

### Compound 9: 6-morpholin-4-ylnaphthalene-1,2-dione

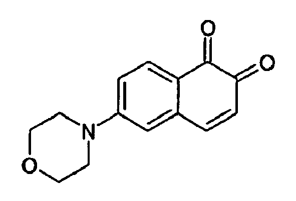

Compound 9 was obtained according to the general procedure III from compound 7, as a red solid (190 mg, 67 %).
¹H-NMR (400 MHz, DMSO): 3.47 (m, 4H, (CH₂)₂-N), 3.74 (m, 4H, (CH₂)₂-O), 6.36 (d, *j* 10.0 Hz, 1H, Ar), 6.96 (dd, *j* 8.9 Hz, *j* 2.6, 1H, Ar), 7.14 (d, *j* 2.6 Hz, 1H, Ar), 7.53 (d, *j* 10.0 Hz, 1H, Ar), 7.80 (d, *j* 8.9 Hz, 1H, Ar).
M/Z (M+H)⁺ = 281.

### Compound 10: 7-(2-bromoethoxy)-2-naphthol

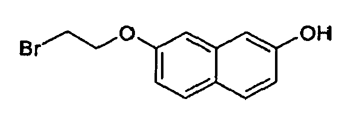

To a mixture of 2,7-dihydroxynaphthalene (160 mg, 1.00 mmol) and K₂CO₃ (300 mg, 2.2 equiv.) in DMF (4 mL) dibromoethane (430 µL, 5.0 equiv.) was added. The heterogeneous mixture was submitted to microwave irradiation. A temperature of 160°C was maintained for 15 minutes. The cooled reaction mixture was hydrolyzed with a 1 N aqueous solution of HCl (40 mL) then extracted with EtOAc (50 mL). The organic layer was washed with brine, dried over MgSO₄ and then was concentrated. The residue was purified by chromatography (eluent CycHex:EtOAc 80:20 - Rf: 0.4). Compound 10 was obtained as an off-white solid (30 mg) in 11 % yield.
¹H-NMR (400 MHz, CDCl₃): 3.72 (t, *j* 6.2 Hz, 2H, CH₂-Br), 4.41 (t, *j* 6.2 Hz, 2H, O-CH₂), 5.10 (bs, 1H, OH), 6.97-7.07 (m, 4H, Ar), 7.68-7.72 (m, 2H, Ar).
M/Z (M+H)⁺ = 267.

### Compound 11: 7-(2-bromoethoxy)naphthalene-1,2-dione

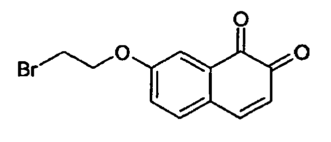

Compound 11 was obtained according to the general procedure IV from compound 10, as a red solid (50 mg, 52 %).
¹H-NMR (400 MHz, DMSO): 3.83 (m, 2H, CH₂-Br), 4.46 (m, 2H, CH₂-O) , 6.25 (d, *j* 10.1 Hz, 1H, Ar), 7.31 (dd, *j* 8.2 Hz, *j* 2.1 Hz, 1H, Ar), 7.42 (d, *j* 2.1 Hz, 1H, Ar), 7.53 (d, *j* 8.2 Hz, 1H, Ar), 7.61 (d, *j* 10.1 Hz, 1H, Ar).
M/Z (M+H)⁺ = 281.

### Compound 12: N-(7-hydroxy-1-naphthyl)acetamide

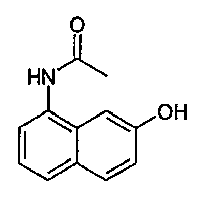

Under inert atmosphere, to a solution of 7-amino-2-naphthol (1.57 mmol) in dichloromethane (8 mL), acetic anhydride (120 µL, 1.0 equiv.) was added. The reaction mixture was stirred 5 Hrs at room temperature, then acetic anhydride (240 µL, 2.0 equiv) was added. The reaction mixture was left at room temperature for 16 Hrs. Solvent was removed then the residue was preabsorbed on silica and was purified by chromatography. Compound 12 was obtained as a brown solid (88 %).
M/Z (M+H)⁺ = 202.

### Compound 13: N-(7,8-dioxo-7,8-dihydronaphthalen-1-yl)acetamide

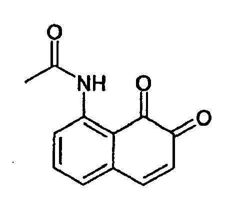

Compound 13 was obtained according to the general procedure IV from compound 12 as a red solid (53 mg, 49 %).
¹H-NMR (400 MHz, CDCl₃): 2.33 (s, 3H, CH₃-CO), 4.75 (bs, 1H, NH), 6.47 (d, *j* 10.0 Hz, 1H, Ar), 7.06 (d, *j* 6.9 Hz, 1H, Ar), 7.44 (d, *j* 10.0 Hz, 1H, Ar), 7.65 (dd, *j* 8.8 Hz, *j* 6.9 Hz, 1H, Ar), 8.87 (d, *j* 8.8 Hz, 1H, Ar).
M/Z (M+H)⁺ = 216.

### Compound 14: tertbutyl 7-hydroxy-1-naphthylcarbamate

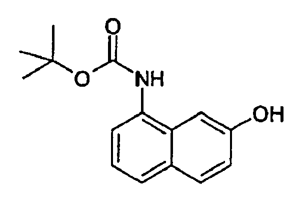

Under inert atmosphere, to a solution of 8-amino-2-naphthol (12.5 mmol) in THF (40 mL), BOC₂O (3.29 g, 1.2 equiv.), then NaHCO₃ (1.27 g, 1.2 equiv) were added. The reaction mixture was refluxed 16 Hrs. Solvent was removed. The residue was hydrolyzed by an aqueous saturated NH₄Cl solution (250 mL), then extracted with EtOAc (300 mL). The organic layer was washed with water (200 mL), brine (200 mL), dried over MgSO₄ and then concentrated. The residue was purified by chromatography (CH₂Cl₂/MeOH 97:03). Compound 14 was obtained as a brown solid (82 %).
¹H-NMR (400 MHz, CDCl₃) : 1.58 (s, 9H, C(CH₃)₃), 5.90 (bs, 1H, OH or NH), 5.62 (bs, 1H, OH or NH), 7.05 (dd, *j* 8.8 Hz, *j* 2.4 Hz, 1H, Ar), 7.17 (d, *j* 2.4 Hz, 1H, Ar), 7.31 (m, 1H, Ar), 7.58 (d, *j* 8.2 Hz, 1H, Ar), 7.71 (m, 2H, Ar).
M/Z (M+H)⁺ = 252.

### Compound 15: tertbutyl 7,8-dioxo-7,8-dihydronaphthalen-1-ylcarbamate

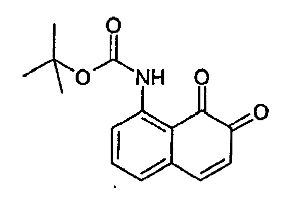

Compound 15 was obtained according to the general procedure IV from compound 14 as a red solid. Compound 15 was contaminated by some selenium derivatives and was used without further purification.
¹H-NMR (400 MHz, CDCl₃) : 1.56 (s, 9H, C(CH₃)₃), 6.46 (d, *j* 10.0 Hz, 1H, Ar), 6.97 (d, *j* 6.8 Hz, 1H, Ar), 7.41 (d, *j* 10.0 Hz, 1H, Ar), 7.61 (m, 1H, Ar), 8.65 (dd, *j* 8.9 Hz, *j* 0.9 Hz, 1H, Ar), 11.29 (bs, 1H, NH).
M/Z (M+H)⁺ = 274.

### Compound 16: N-(7-hydroxy-1-naphthyl)ethanesulfonamide

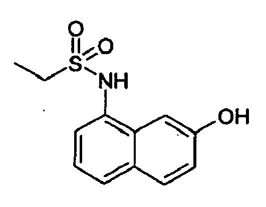

Under inert atmosphere, to a solution of 8-amino-2-naphthol (3.1 mmol) in THF (15 mL), triethylamine (660 µL, 1.5 equiv.), then ethylsulfonylchloride (1.1 equiv.) were added carefully. The reaction mixture was stirred 1 Hr at room temperature, then the solvent was removed. The residue was hydrolyzed by an aqueous saturated NH₄Cl solution (100 mL), and then extracted with EtOAc (100 mL). The organic layer was washed with water (50 mL), brine (50 mL), dried over MgSO₄ then concentrated. The residue was purified by chromatography (CH₂Cl₂/MeOH 100:0 to 98:02). Compound 16 was obtained as an off-white solid in quantitative yield.
¹H-NMR (400 MHz, CDCl₃) : 1.59 (t, *j* 7.4 Hz, 3H, SO₂-CH₂- CH₃), 3.34 (q, *j* 7.4 Hz, 2H, SO₂-CH₂- CH₃), 4.15 (bs, 2H, OH, NH), 6.85 (m, 1H, Ar), 7.34 (m, 2H, Ar), 7.38 (dd, *j* 8.9 Hz, *j* 2.4 Hz, 1H, Ar), 7.75 (d, *j* 2.4 Hz, 1H, Ar), 7.85 (d, *j* 8.9 Hz, 1H, Ar).
M/Z (M+H)⁺ = 252.

### General procedure VIa: semicarbazone formation from 1,2-naphthoquinone in presence of semicarbazide hydrochloride.

To a suspension of 1-2-naphthoquinone derivative (1.0 mmol) in a solvent (10 mL), semicarbazide hydrochloride (110 mg 1.0 equiv.) was added. The reaction mixture was stirred 15 minutes at room temperature and then was filtered off. The solid was washed with solvent (3x2 mL) Et₂O (5x2 mL), then was dried under reduced pressure at 50°C for 15 Hrs.

The following examples of compounds of general formula (I) were obtained using above described routes of synthesis.

### Example 1: 4-morpholin-4-ylnaphthalene-1,2-dione 2-semicarbazone

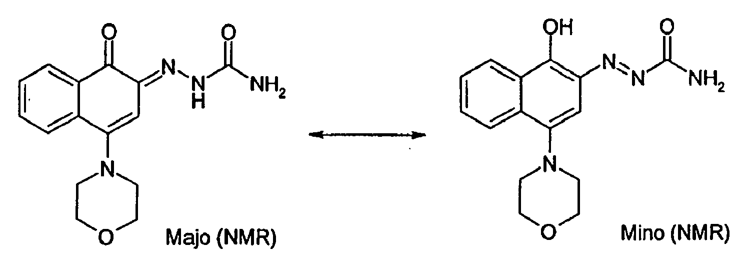

Example 1 was obtained according to the general procedure VIa from compound 1, using EtOH as a solvent, as an orange solid. Further purification was achieved through various precipitations and triturations.
¹H-NMR (400 MHz, DMSO) tautomer mixture at 50°C (75:25):
Major tautomer: 2.99 (m, 4H, (CH₂)₂-N), 3.84 (m, 4H, (CH₂)₂-O), 6.40 (s, 1H, Ar), 7.13 (br, 2H, NH₂), 7.58 (m, 1H, Ar), 7.80-7.89 (m, 2H, Ar), 8.27 (m, 1H, Ar), 13.36 (s, 1H, NH).
Minor tautomer: 3.04 (m, 4H, (CH₂)₂-N), 3.84 (m, 4H, (CH₂)₂-O), 6.58 (br, 2H, NH₂), 6.72 (s, 1H, Ar), 7.54 (m, 1H, Ar), 7.74 (m, 2H, Ar), 8.07 (d, *j* 8.1 Hz, 1H, Ar), 10.80 (s, 1H, OH).
M/Z (M+H)⁺ = 301.

### Example 2: 4-(4-methylpiperazin-1-yl)naphthalene-1,2-dione 2-semicarbazone, HCl salt

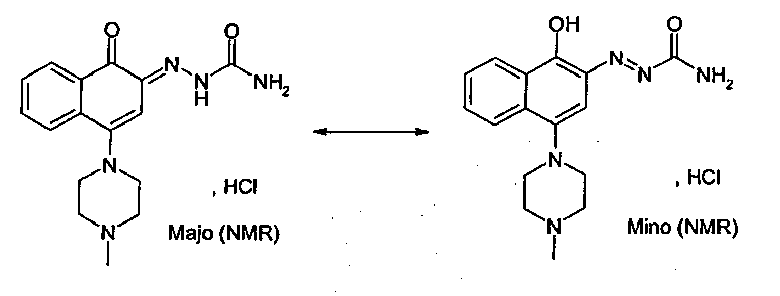

The free base of example 2 was obtained according to the general procedure VIa from compound 2, using EtOH as a solvent, as an orange solid. To the free base in suspension in MeOH, concentrated aqueous HCl was added. The suspension was stirred 45 minutes, and then was filtered off. Solid was washed with MeOH (2x2 mL), and then was dried under reduced pressure at 50°C for 15 Hrs. Example 2 was obtained as an orange solid.
¹H-NMR (400 MHz, DMSO) tautomer mixture at 25°C (75:25):
Major tautomer: 2.85 (s, 3H, (CH₃)-N), 3.12-3.51 (partially under HOD signal) (m, 8H, 2 x (CH₂)₂-N), 6.46 (s, 1H, Ar), 7.35 (br, 2H, NH₂), 7.61 (m, 1H, Ar), 7.84 (m, 2H, Ar), 8.25 (m, 1H, Ar), 10.98 (bs, 1H, HCl salt) 13.42 (s, 1H, NH).
Minor tautomer: 2.85 (s, 3H, (CH₃)-N), 3.12-3.51 (partially under HOD signal) (m, 8H, 2 x (CH₂)₂-N), 6.87 (br, 2H, NH₂), 7.61 (m, 1H, Ar), 7.72 (m, 2H, Ar), 8.08 (m, 1H, Ar), 11.09 (s, 1H, OH), 1 aromatic signal is missing.
M/Z (M+H)⁺ = 314.

### Example 3: 6-methoxynaphthalene-1,2-dione 2-semicarbazone

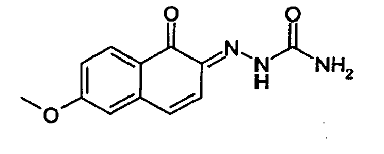

Example 3 was obtained according to the general procedure VIa from compound 3, using MeOH as solvent, as a brown solid.

Further purification was realized through precipitation in MeOH.
¹H-NMR (400 MHz, DMSO): 3.92 (s, 3H, CH₃-O), 6.81 (d, *j* 9.4 Hz, 1H, Ar), 7.05 (d, *j* 9.4 Hz, 1H, Ar), 7.07 (dd, *j* 8.8 Hz, *j* 2.5 Hz, 1H, Ar), 7.18 (d, *j* 2.5 Hz, 1H, Ar), 8.14 (bs, 2H, NH₂), 8.14 (d, *j* 8. 8 Hz, 1H, Ar), 13.81 (s, 1H, NH).
M/Z (M+H)⁺ = 246.

### Exemple 4: 6-morpholin-4-ylnaphthalene-1,2-dione 2-semicarbazone

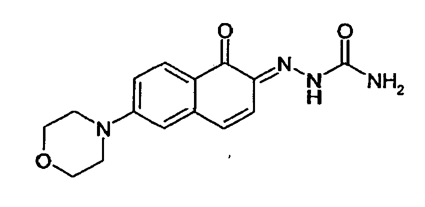

Example 4 was obtained according to the general procedure VIa from compound 9, using MeOH as a solvent, as a dark red solid.
¹H-NMR (400 MHz, DMSO): 3.44 (m, 4H, (CH₂)₂-N), 3.75 (m, 4H, (CH₂)₂-O), 6.72 (d, *j* 9.6 Hz, 1H, Ar), 6.96 (d, *j* 9.6 Hz, 1H, Ar), 7.06 (m, 2H, Ar), 7.20-7.40 (br, 2H, NH₂), 8.01 (d, *j* 8.6 Hz, 1H, Ar), 13.87 (s, 1H, NH).
M/Z (M+H)⁺ = 301.

### General procedure VIb: semicarbazone formation from 1,2-naphthoquinone in presence of semicarbazide hydrochloride in DMF.

To a suspension of 1-2-naphthoquinone derivative (1.0 mmol) in DMF (10 mL), semicarbazide hydrochloride (110 mg 1.0 equiv.) was added. The reaction mixture was stirred 15 minutes at room temperature and then water (30 mL) was added. A precipitate was obtained. The solid was filtered off, washed with water (2x5 mL), CH₃CN (2x2mL), Et₂O (5x2 mL), then was dried under reduced pressure at 50°C for 15 Hrs.

### Example 5: 6-bromonaphthalene-1,2-dione 2-semicarbazone

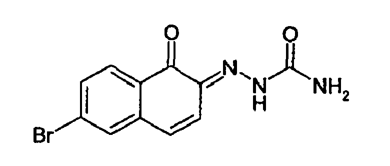

¹H-NMR (400 MHz, DMSO): 6.91 (d, *j* 9.6 Hz, 1H, Ar), 7.10 (d, *j* 9.6 Hz, 1H, Ar), 7.3 -7.70 (bs, 2H, NH₂), 7.71 (dd, *j* 8.4 Hz, *j* 1.9 Hz, 1H, Ar), 7.97 (s, 1H, Ar), 8.10 (d, *j* 8.4, 1H, Ar), 13.75 (s, 1H, NH).
M/Z (M+H)⁺ = 294.

### Example 6: 4-methylnaphthalene-1,2-dione 2-semicarbazone

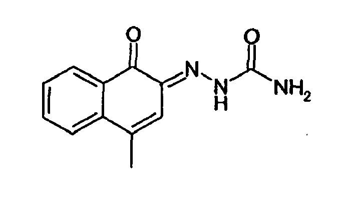

Example 6 was obtained according to the general procedure VIb from compound 4, as an orange solid.
¹H-NMR (400 MHz, DMSO): 2.34 (s, 3H, CH₃), 6.71 (s, 1H, Ar), 7.37 (bs, 2H, NH₂), 7.57 (t, *j* 7.6 Hz, 1H, Ar), 7.70 (d, *j* 7.8 Hz, 1H, Ar), 7.82 (t, *j* 7.6 Hz, 1H, Ar), 8.24 (d, *j* 7.8 Hz, 1H, Ar), 13.74 (s, 1H, NH).
M/Z (M+H)⁺ = 230.

### Example 7: 7-bromonaphthalene-1,2-dione 2-semicarbazone

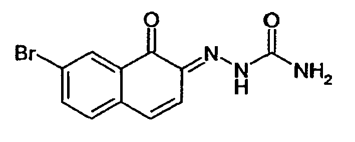

Example 7 was obtained according to the general procedure VIb from compound 5, as a brown solid.
¹H-NMR (400 MHz, DMSO): 6.90 (d, *j* 9.7 Hz, 1H, Ar), 7.12 (d, *j* 9.7 Hz, 1H, Ar), 7.30 -7.70 (bs, 2H, NH₂), 7.64 (d, *j* 8.3 Hz, 1H, Ar), 7.94 (dd, *j* 8.3 Hz, *j* 2.1 Hz, 1H, Ar), 8.25 (d, *j* 2.1, 1H, Ar), 13.70 (s, 1H, NH).
M/Z (M+H)⁺ = 294.

### Example 8: 7-(2-bromoethoxy)naphthalene-1,2-dione 2-semicarbazone

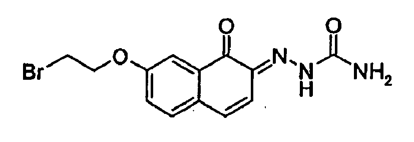

Example 8 was obtained according to the general procedure VIb from compound 11, as a brown solid. It should be noted that example 8, in this condition, was contaminated by the chloro analogue. A bromo chloro exchange was observed during the reaction (10 - 15 %). Compounds were not separated.
¹H-NMR (400 MHz, DMSO): 3.84 (m, 2H, CH₂-Br), 4.46 (m, 2H, CH₂-O), 6.72 (d, *j* 9.7 Hz, 1H, Ar), 7.07 (d, *j* 9.7 Hz, 1H, Ar), 7.30 -7.60 (bs, 2H, NH₂), 7.41 (dd, *j* 8.3 Hz, j 2.3, 1H, Ar), 7.61 (m, 2H, Ar), 13.73 (s, 1H, NH).
M/Z (M+H)⁺ = 338.

### Example 9: N-{-7-[(aminocarbonyl)hydrazono]-8-oxo-7,8-dihydronaphthalen-1-yl}acetamide

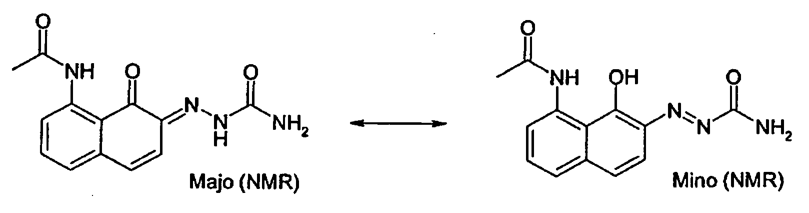

Example 9 was obtained according to the general procedure VIb from compound 13, as an orange solid.
¹H-NMR (400 MHz, DMSO) tautomer mixture at 25°C (85:15):
Major tautomer: 2.25 (s, 3H, CH₃-N), 6.86 (d, *j* 9.7 Hz, 1H, Ar), 6.09 (d, *j* 9.7 Hz, 1H, Ar), 7.31 (m, 1H, Ar), 7.35-7.65 (br, 2H, NH₂), 7.74 (t, *j* 8.0 Hz, 1H, Ar), 8.62 (m, 1H, Ar), 12.17 (s, 1H, NH), 13.50 (s, 1H, NH).
Minor tautomer: : 2.20 (s, 3H, CH₃-N), 7.04 (d, *j* 10.1 Hz, 1H, Ar), 7.21 (m, H, Ar), 7.38 (d, *j* 10.1 Hz, 1H, Ar), 7.35-7.65 (br, 2H, NH₂), 7.66 (t, *j* 7.9 Hz, 1H, Ar), 11.32 (s, 1H, NH), 12.48 (s, 1H, NH), 1 proton is missing.
M/Z (M+H)⁺ = 273.

### Example 10: tert-butyl-7-[(aminocarbonyl)hydrazono]-8-oxo-7,8-dihydronaphthalen-1-ylcarbamate

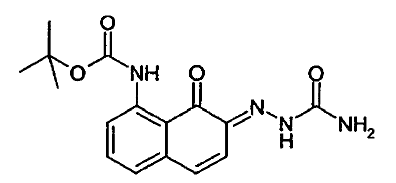

Example 10 was obtained according to the general procedure VIb from compound 15, as an orange solid.
¹H-NMR (400 MHz, CDCl₃) : 1.59 (s, 9H, C(CH₃)₃), 6.71 (d, *j* 9.7 Hz, 1H, Ar), 6.91 (d, *j* 9.7 Hz, 1H, Ar), 7.02 (d, *j* 7.1 Hz, 1H, Ar), 7.61 (m, 3H, Ar + NH₂), 8.52 (d, *j* 8.6 Hz, 1H, Ar), 11.63 (s, 1H, NH), 13.70 (s, 1H, NH).
M/Z (M+H)⁺ = 331.

### Example 11: 8-aminonaphthalene-1,2-dione 2-semicarhazone , hydrochloride salt

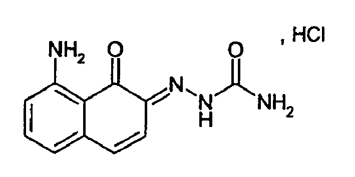

Under inert atmosphere, to a suspension of example 10 (10.6 mmol) in MeOH (150 mL), HCl 2N solution in Et₂O (10 equiv.) was added. The reaction mixture was stirred at room temperature for 16 Hrs. Solvents were removed. The residue was taken in Et₂O (10 mL). The resulting solid was filtered off, washed with Et₂O (2x4 mL) and dried under reduced pressure at 50°C for 15 Hrs. Example 11 was obtained as a brown solid.
¹H-NMR (400 MHz, DMSO): 6.65 (m, 2H, Ar), 6.78 (d, *j* 8.4 Hz, 1H, Ar), 6.89 (d, *j* 9.6 Hz, 1H, Ar), 7.18 (bs, 2H, NH₂), 7.35 (dd, *j* 8.4 Hz, *j* 7.3 Hz, 1H, Ar), 7.50-8.50 (bs, 3H, NH₃⁺), 13.54 (s, 1H, NH).
M/Z (M+H)⁺ = 231.

### General procedure VII: formation of amide from amino semicarbazone.

Under inert atmosphere, to a mixture of aminosemicarbazone derivative (1.2 equiv) and a carboxylic acid (0.16 mmol) in pyridine (4 mL), cooled by an ice bath, phosphorous oxychloride (0.22 mmol, 1.4 equiv.) was added carefully. The reaction mixture was stirred 1 Hr at room temperature, then cooled with an ice bath. An excess of phosphorus oxychloride (0.44 mmol, 2.8 equiv.) was added. The reaction mixture was stirred 15 minutes at room temperature. Solvent was removed. The residue was hydrolyzed by an aqueous 1 N sodium hydroxide solution (30 mL), and then extracted with EtOAc (50 mL). The organic layer was washed with brine (25 mL), dried over MgSO₄ and concentrated.

### Example 12: N-{7-[(aminocarbonyl)hydrazono]-8-oxo-7,8-dihydronaphthalen-1-yl}-3-piperidin-1-ylpropanamide trifluoroaceticacid salt

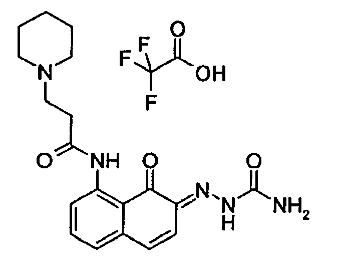

Example 12 was obtained according to the general procedure VII from example 11 with 1-piperidinepropionic acid, and purified by preparative HPLC.
M/Z (M+H)⁺ = 370.

### General procedure VIII: Nucleophilic displacement of bromoalkyl semicarbazone derivatives.

To a solution of bromoalkyl semicarbazone derivatives (0.22 mmol) in DMF (1 mL), a nucleophile (4.0 equiv) was added dropwise. The reaction mixture was stirred at room temperature for 48 Hrs. The reaction mixture was hydrolyzed by water (5 mL). A precipitate was obtained. The solid was filtered off, washed with water (2x1 mL), Et₂O (3x2mL), and dried under reduced pressure at 50°C for 15 Hrs.

### Example 13: 7-(2-morpholin-4-yl-ethoxy) naphtalene-1,2-dione2-semicarbazone

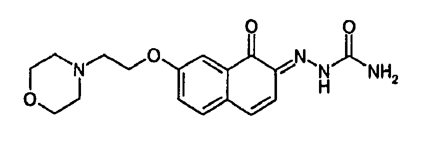

Example 13 was obtained according to the general procedure VIII from example 8 and morpholine, as a red solid.
¹H-NMR (400 MHz, DMSO) : 2.72 (br, 2H, CH₂-N), 3.58 (br, 4H, (CH₂)₂-O), 4.21 (br, 2H, CH₂-O), 6.69 (d, *j* 9.4 Hz, 1H, Ar), 7.05 (m, *j* 9.4 Hz, 1H, Ar), 7.37 (m, 1H, Ar), 7.4 (br, 2H, NH₂), 7.58 (m, 1H, Ar), 7.65 (s, 1H, Ar), 13.72 (br, 1H, NH) signals for 4 protons are missing.
M/Z (M+H)⁺ = 345.

The HCl salt of example 13 was also obtained.

Example 13 was taken in a 1.25 N methanolic solution of HCl (5.0 equiv.). The reaction mixture was stirred at room temperature for 1 hour, and then the precipitate was filtered, washed with Et₂O, and dried under reduced pressure at 50°C for 15 Hrs. The HCl salt of example 13 was obtained as an orange solid.
¹H-NMR (400 MHz, DMSO): 3.17 (br, 2H, CH₂-N), 3.54 (br, 2H, CH₂-N), 3.60 (br, 2H, CH₂-N), 3.77 (br, 2H, CH₂-O), 3.96 (br, 2H, CH₂-O), 4.52 (br, 2H, CH₂-O), 6.73 (d, *J* 9.7 Hz, 1H, Ar), 7.09 (d, *J* 9.7 Hz, 1H, Ar), 7.30-7.60 (bs, 2H, NH₂), 7.44 (dd, *J* 8.6 Hz, *J* 2.6 Hz, 1H, Ar), 7.64 (d, *J* 8.6 Hz, 2H, NH₂), 7.72 (d, *J* 2.6 Hz, 1H, Ar), 10.60 (bs, 1H, NH), 13.71 (s, 1H, NH).
M/Z (M+H)⁺ = 345.
mp = 231-234°C.

### Example 14: 7-(2-dimethylamino-ethoxy)naphtalene-1,2-dione-2-semicarbazone, trifluoroaceticacid salt

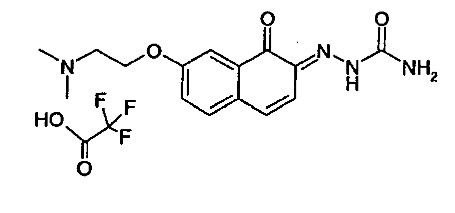

Example 14 was obtained according to the general procedure VIII from example 8 with dimethylamine, as a red solid as a trifluoroacetic acid salt after purification by preparative HPLC.
¹H-NMR (400 MHz, DMSO) : 2.87 (s, 6H, CH₃-N), 3.55 (s, 2H, CH₂-N), 4.46 (s, 2H, CH₂-O), 6.73 (d, *J* 9.5 Hz, 1H, Ar), 7.08 (d, *J* 9.5 Hz, 1H, Ar), 7.42 (m, 3H, Ar + NH₂), 7.66 (m, 2H, Ar), 13.70 (s, 1H, NH).
M/Z (M+H)⁺ = 303.
mp = 167-171°C.

### Example 15: 4-bromonaphthalene-1,2-dione 2-semicarbazone

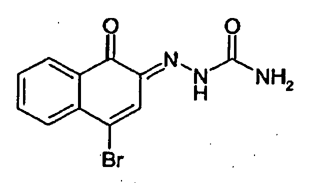

To a suspension of naphthalene-1,2-dione 2-semicarbazone (1.07 g, 4.9 mmol) in AcOH (30 mL), bromine (280 µL, 1.1 equiv.) was added dropwise. After 3 Hrs at room temperature, the orange solution was diluted with EtOAc (to 300 mL), then was washed successively by water (200 mL), a saturated thiosulfate solution (200 mL), water (200 mL), a saturated NaHCO₃ solution (200 mL) and brine (100 mL). The organic layer was dried over MgSO₄.

A yellow precipitate was formed in the organic layer after a few hours. Precipitate was collected, washed with Et₂O (3x5 mL) and dried under reduced pressure for 15 Hrs. A yellow solid was obtained in 62 % yield.
¹H-NMR (400 MHz, DMSO): 7.31 (s, 1H, Ar), 7.40-7.60 (bs, 2H, NH₂), 6.67 (m, 1H, Ar), 7.90 (m, 2H, Ar), 8.27 (dd, *j* 7.7 Hz, *j* 0.9 Hz, 1H, Ar), 13.58 (s, 1H, NH).
M/Z (M+H)⁺ = 294.

### General procedure IX: Benzylic bromination and in situ nucleophilic displacement of semicarbazone derivatives.

Under inert atmosphere, to a solution of methylnaphthalene-1,2-dione 2-semicarbazone (0.22 mmol) in CHCl₃ (3 mL), bromine (13 µL, 1.2 equiv) was added. The reaction mixture was subjected to microwave irradiation. A temperature of 160°C was maintained for 20 minutes. To the cooled reaction mixture, nucleophilic species (25 equiv) was added. The resulting solution was subjected to microwave irradiation. A temperature of 130°C was maintained for 5 minutes. The solvent was evaporated. The residue was taken in Et₂O (2 mL). The obtained precipitate was filtered off, washed with Et₂O (2x2 mL) and then purified by preparative HPLC.

### Example 16: 4-(morpholin-4-ylmethyl)naphthalene-1,2-dione2-semicarbazone, trifluoroacetic acid salt

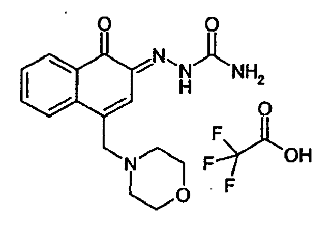

Example 16 was obtained according to the general procedure IX from example 6, using morpholine as nucleophilic species, as an orange solid.
¹H-NMR (400 MHz, DMSO): 3.25 (br, 2H, CH₂), 3.62-3.75 (br, 4H, CH₂), 3.96 (br, 2H, CH₂), 4.57 (br, 2H, CH₂), 7.18 (s, 1H, Ar), 7.52 (br, 2H, NH₂), 7.63 (s, 1H, Ar), 7.87 (s, 1H, Ar), 8.03 (s, 1H, Ar), 8.30 (s, 1H, Ar), 9.80 (br, 1H, TFA salt), 13.93 (br, 1H, NH).
M/Z (M+H)⁺ = 315.
mp = 206-210°C.

### Example 17: 4-(ethylamine-4-ylmethyl)naphthalene-1,2-dione2-semicarbazone, trifluoroacetic acid salt

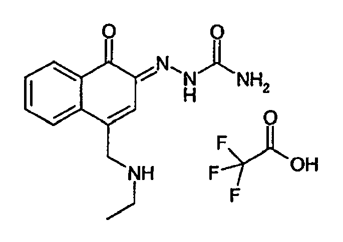

Example 17 was obtained according to the general procedure IX from example 6, using ethylamine as nucleophilic species, as an orange solid.
¹H-NMR (400 MHz, DMSO) : 1.26 (m, 3H, CH₃), 3.13 (br, 2H, CH₂-N), 3.37 (br, 2H, CH₂-N), 7.10 (s, 1H, Ar), 7.53 (br, 2H, NH₂), 7.65 (m, 1H, Ar), 7.89 (m, 1H, Ar), 8.31 (d, *j* 7.8 Hz, 1H, Ar), 8.85 (br, 2H, NH + TFA salt), 13.90 (s, 1H, NH).
M/Z (M+H)⁺ = 273.

### Example 18: 4-(hydroxymethy)naphthalene-1,2-dione 2-semicarbazone

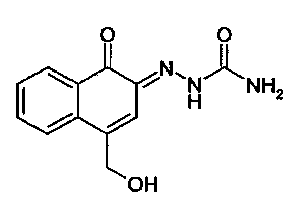

Example 18 was obtained according to the general procedure IX from example 6, using water as nucleophilic species, as a brown solid.
¹H-NMR (400 MHz, DMSO): 4.65 (s, 2H, CH₂-OH), 5.36 (br, 1H, OH), 6.93 (s, 1H, Ar), 7.50 (br, 2H, NH₂), 7.56 (t, *j* 7.4 Hz, 1H, Ar), 7.74 (d, *j* 7.8 Hz, 1H, Ar), 7.80 (t, *j* 7.0 Hz, 1H, Ar), 8.24 (d, *j* 7.4 Hz, 1H, Ar), 13.64 (s, 1H, NH).
M/Z (M+H)⁺ = 246.

### Example 19: 4-(methylhydroxymethy)naphthalene-1,2-dione 2-semicarbazone

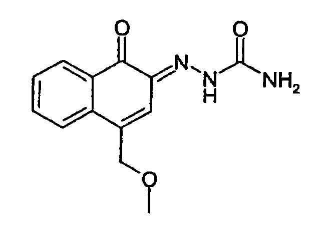

Example 19 was obtained according to the general procedure IX from example 6 and, using methanol as nucleophilic species. It should be noted that example 19 was not purified by preparative HPLC but by standard chromatography (CH₂Cl₂ to 1% MeOH/CH₂Cl₂). Example 19 was obtained as an orange solid.
¹H-NMR (400 MHz, DMSO): 3.37 (s, 3H, CH₃-O), 4.57 (s, 2H, CH₂-O), 6.90 (s, 1H, Ar), 7.47 (br, 2H, NH₂), 7.59 (t, *j* 7.4 Hz, 1H, Ar), 7.74 (t, *j* 7.8 Hz, 1H, Ar), 7.80 (t, *j* 7.5 Hz, 1H, Ar), 8.25 (d, *j* 7.7 Hz, 1H, Ar),13.69 (s, 1H, NH).
M/Z (M+H)⁺ = 260.

### Example 20: Effect of example 13 on CSF-glutamate level

For this example, intracerebroventricular retrodialysis was performed on rats according to techniques known to those skilled in the art. The analysis of microdialysates was performed according to Israël and collaborators' publication (Neurochem Int 22(1): 53-58, 1993). Procedures are described below:

### Intracerebroventricular retrodialysis in rats:

Surgery was performed on adult male Sprague-Dawley rats weighing 330-340g at the time of surgery. Following induction of anaesthesia with intraperitoneal administration of ketamine/xylazine, animals were secured in a stereotaxic frame in the flat-skull position at a constant setting of -3.3mm between ear and incisor bars. After defining the "stereotaxic zero" in accordance with Paxinos and Watson's atlas (*1986*), a microdialysis guide cannula was directed towards the right lateral ventricle with the coordinates from Bregma: anteroposterior -0.8mm, mediolateral +1.5mm, and dorsoventral (DV) -1.5mm (CMA12 probe: 2mm membrane) (Khandewal et al., J Neurosci Methods 133(1-2): 181-189, 2004).

The guide cannula was fixed to the skull using dental cement and three stainless steel screws. The assembly was protected by a tube (screw-cap tubes Eppendorf).

A plastic collar allowed each animal to be tethered to a Swivel assembly of the CMA awake animal system.

Following surgery, rats were individually housed and were provided food and water *ad libitum.* Animals were allowed 3-4 days of post-operative recovery time.

### In vivo microdialysis

Dialysis probes (CMA12/2mm, 500µm membrane outside diameter) were purchased from CMA/Microdialysis (Stockholm, Sweden) and were equilibrated according to manufacturer's specifications the day before implantation.

All experiments were performed between 8.00 am and 6.00 pm. The prepared probe was implanted via the guide cannula into the right lateral ventricle and perfused with artificial cerebrospinal fluid (aCSF: 145mM NaCl, 2.70mM KCl, 1.20mM CaCl₂, 1.0mM MgCl₂, 0.45mM NaH₂PO4 and 2.33mM Na₂HPO₄, pH 7.4) at a flow rate of 1µL/min.

After the probe was inserted into the animal, a 1h stabilization period was allowed after which time dialysate sampling was conducted in the unrestrained rat. For the next 2h, fractions were then collected at 20min intervals into plastic vials in order to estimate the physiological release of the intraventricular CSF glutamate content. Baseline conditions were reached when 3 successive effluent fractions gave similar glutamate contents. Animals were then treated for 40min by retrodialysis without stopping perfusion using 30µM solution of the test compound and dialysis samples were still collected every 20min. After the retrodialysis, dialysis samples were collected for a total period of 4h. The collected fractions (20µL) were immediately, transferred into liquid nitrogen (-180°C) and then kept frozen (-80°C) until further analysis.

### Histology

At the end of each experiment, rats were euthanized and brains were quickly removed and stored in buffered formalin (30% (v/v) formaldehyde/water) for 24h. The correct placement of the microdialysis probe was checked by examination of the coronal sections under a stereomicroscope.

Animals with incorrect probe placement were excluded from the study.

### Analysis of microdialysates

Samples were subsequently analysed for glutamate determination using an enzymatic assay (Israël et al., 1993). The procedure is based on the oxidation of L-glutamate dehydrogenase with subsequent NADH production, the NADH being continuously evaluated using the photobacterium chemiluminescent reaction at 460-490nm.

Microdialysates were extemporaneously thawed and maintained on ice. In a 96-well plate samples were diluted (1:2.5 or 1:5; final volume: 10µL) in assay buffer (50mM NaH₂PO₄ / Na₂HPO₄, pH=7.1-7.2) and the final volume was adjusted to 250µL with assay buffer.

The final enzymatic mix was prepared by adding 550µL of freshly prepared 0.05% decanal (0.05% decanal in 50mM NaH₂PO₄ / Na₂HPO₄, pH=7.4) to 5560µL of enzymatic mix (348U/mL GIDH, 3.59U/mL NAD(P)H:FMN oxidoreductase, 3.59mg/mL Luciferase, 2.98mg/mL β-NAD prepared in 0.2M tris pH=8.0, 5.6µg/mL FMN, and which activity was tested (50µL sample) using a Multiway Analyser (Statice, Besançon, France)) and diluted in assay buffer (1:45). The final enzymatic solution was kept at RT for 15-20min before using in order to allow signal stabilization.

40µL of final containing decanal was added to samples allowing a light emission directly proportional to the quantity of glutamate in each sample. The luminescence was read at 460-490nm.

The assay was performed on a luminescence imaging system with charge coupled device camera CyBi™-Lumax D, using flash-luminescence readout.

The test samples were determined from a standard curve (0 to 25pmol of a standard solution of 1M glutamic acid in osmosed water) prepared in assay buffer in the same conditions. If samples were not enough concentrated, a second run was performed using a standard curve from 0 to 7.5pmol.

### Data analysis

The basal glutamate level for each rat was estimated by averaging the last 3 samples collected before administration of the drug. In figure 1, glutamate levels were expressed as a percentage of the baseline arbitrarily set at 100%.

### Results

One rat out of five (rat C) did not response to the compound of Example 13.

Four rats (rats A, B, D and E) showed a decrease up to 50% of the cerebrospinal fluid glutamate level (after 80 minutes) after intracerebroventricular injection of the compound of Example 13.

## Claims

1. A compound of general formula (I): 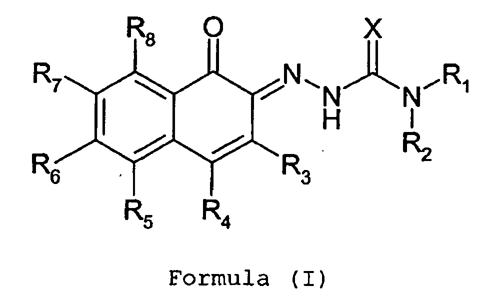 wherein:
**X** is S or O;
**R₁** and **R₂** are independently from each other hydrogen, alkyl, or can be linked to generate heterocycloalkyl;
**R₃, R₅, R₆, R₇** and **R₈** are independently from each other hydrogen, halogen, CN, CF₃, OCF₃, alkyl, COOH, CONH(alkyl), CON(alkyl)(alkyl), CO(heterocycloalkyl), OH, O-(alkyl), S-(alkyl), NH₂, NH(alkyl), N- (alkyl) (alkyl), NHCO (alkyl), N(Alkyl)CO(alkyl), NHSO₂(alkyl), N(Alkyl)SO₂(alkyl), heterocycloalkyl, aryl or heteroaryl;
**R₄** is hydrogen, halogen, CN, CF₃, OCF₃, alkyl, COOH, CONH(alkyl), CON(alkyl)(alkyl), CO(heterocycloalkyl), O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl;
With the proviso that **R₁, R₂, R₃, R₄, R₅, R₆, R₇** and **R₈** be not hydrogen all together;
The two Adjacent R groups **R₃-R₄, R₄-R₅, R₅-R₆, R₆-R₇** and **R₇-R₈** can be linked to generate a 5 to 8 member ring with atoms selected from C, N, O, S and P;
as well as pharmaceutically acceptable salts thereof, a prodrug of the compound or the salt, or a solvate or hydrate of the compound, the salt or the prodrug.

2. Compounds according to claim 1, of formula (II): 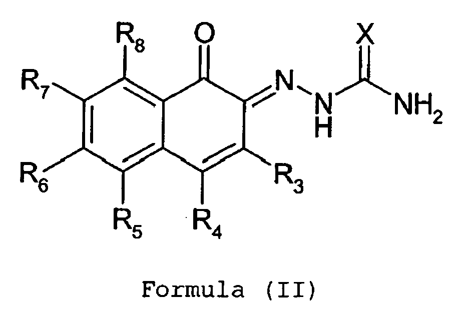 wherein:
**X** is S or O;
**R₃, R₅, R₆, R₇** and **R₈** are independently from each other hydrogen, halogen, CN, CF₃, OCF₃, alkyl, COOH, CONH(alkyl), CON (alkyl) (alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl.
**R₄** is hydrogen, halogen, CN, CF₃, OCF₃, alkyl, COOH, CONH(alkyl), CON(alkyl)(alkyl), CO(heterocycloalkyl), O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl;
With the proviso that **R₃, R₄, R₅, R₆, R₇** and **R₈** be not hydrogen all together;
The two Adjacent R groups **R₃-R₄, R₄-R₅, R₅-R₆, R₆-R₇** and **R₇-R₈** can be linked to generate a 5 to 8 member ring with atoms selected from C, N, O, S and P;
as well as pharmaceutically acceptable salts thereof, a prodrug of the compound or the salt, or a solvate or hydrate of the compound, the salt or the prodrug.

3. Compounds according to claim 1, of formula (III): 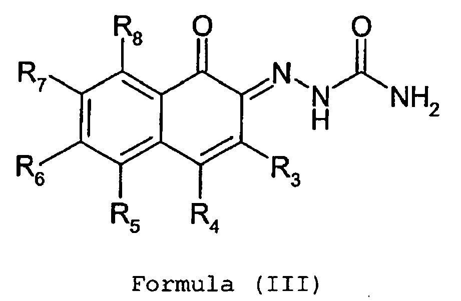 wherein:
**R₃, R₅, R₆, R₇** and **R₈** are independently from each other hydrogen, halogen, CN, CF₃, OCF₃, alkyl, COOH, CONH(alkyl), CON(alkyl)(alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl.
**R₄** is hydrogen, halogen, CN, CF₃, OCF₃, alkyl, COOH, CONH(alkyl), CON(alkyl)(alkyl), CO(heterocycloalkyl), O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl;
With the proviso that **R₃, R₄, R₅, R₆, R₇** and **R₈** be not hydrogen all together;
The two Adjacent R groups **R₃-R₄, R₄-R₅, R₅-R₆, R₆-R₇** and **R₇-R₈** can be linked to generate a 5 to 8 member ring with atoms selected from C, N, O, S and P;
as well as pharmaceutically acceptable salts thereof, a prodrug of the compound or the salt, or a solvate or hydrate of the compound, the salt or the prodrug.

4. Compounds according to claim 3, wherein:
**R₃, R₅, R₆, R₇** and **R₈** is hydrogen;
**R₄** is halogen, CN, CF₃, OCF₃, alkyl, COOH, CONH(alkyl), CON(alkyl)(alkyl), CO(heterocycloalkyl), O-(alkyl), S-(alkyl), NH₂, NH(alkyl), N-(alkyl) (alkyl), NHCO(alkyl), N (Alkyl)CO(alkyl), NHSO₂(alkyl), N(Alkyl)SO₂(alkyl), heterocycloalkyl, aryl or heteroaryl;
as well as pharmaceutically acceptable salt thereof, a prodrug of the compound or the salt, or a solvate or hydrate of the compound, the salt or the prodrug.

5. Compounds according to claim 3, wherein:
**R₃, R₄, R₅, R₇** and **R₈** is hydrogen;
**R₆** is halogen, CN, CF₃, OCF₃, alkyl, COOH, CONH (alkyl), CON(alkyl)(alkyl), CO(heterocycloalkyl), OH, O-(alkyl), S-(alkyl), NH₂, NH(alkyl), N- (alkyl) (alkyl), NHCO (alkyl), N(Alkyl)CO(alkyl), NHSO₂(alkyl), N(Alkyl)SO₂(alkyl), heterocycloalkyl, aryl or heteroaryl;
as well as pharmaceutically acceptable salt thereof, a prodrug of the compound or the salt, or a solvate or hydrate of the compound, the salt or the prodrug.

6. Compounds according to claim 3, wherein:
**R₃, R₄, R₅, R₆** and **R₈** is hydrogen;
**R₇** is halogen, CN, CF₃, OCF₃, alkyl, COOH, CONH (alkyl), CON (alkyl) (alkyl), CO(heterocycloalkyl), OH, O-(alkyl), S-(alkyl), NH₂, NH(alkyl), N-(alkyl)(alkyl), NHCO(alkyl), N(Alkyl)CO(alkyl), NHSO₂(alkyl), N(Alkyl)SO₂(alkyl), heterocycloalkyl, aryl or heteroaryl;
as well as pharmaceutically acceptable salt thereof, a prodrug of the compound or the salt, or a solvate or hydrate of the compound, the salt or the prodrug.

7. Compounds according to claim 3, wherein:
**R₃, R₄, R₅, R₆** and **R₇** is hydrogen;
**R₈** is halogen, CN, CF₃, OCF₃, alkyl, COOH, CONH(alkyl), CON(alkyl)(alkyl), CO(heterocycloalkyl), OH, O-(alkyl), S-(alkyl), NH₂, NH(alkyl), N- (alkyl)(alkyl), NHCO (alkyl), N(Alkyl)CO(alkyl), NHSO₂(alkyl), N(Alkyl)SO₂(alkyl), heterocycloalkyl, aryl or heteroaryl;
as well as pharmaceutically acceptable salt thereof, a prodrug of the compound or the salt, or a solvate or hydrate of the compound, the salt or the prodrug.

8. Use of a compound of formula (I) according to claim 1 in the manufacture of a drug for use in the treatment and/or prophylaxis of conditions and diseases linked to abnormalities in glutamatergic transmission.

9. Use according to claim 8, where the condition or disease linked to abnormalities in glutamatergic transmission is selected from epilepsy, dementias (including dementias of the Alzheimer's type, vascular dementias, AIDS-dementia complex), parkinsonism and movement disorders (including Parkinson's disease, Huntington's disease, dystonias, Gilles de la Tourette syndrome, dyskinesias), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis), headache, migraine, myoneural disorders (myasthenia gravis, acute muscle spasms, myopathies), disorders of the eye and visual pathways, intracranial trauma/injury, trauma/injury to nerves and spinal cord, poisoning and toxic effects of non medicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, restless legs syndrome, endocrine and metabolic diseases (diabetes, hypoglycaemia), acute and chronic pain; nausea and vomiting; irritable bowel syndrome.

10. A pharmaceutical composition comprising a pharmaceutically effective amount of a compound of the formula (I) according to claim 1, or pharmaceutically acceptable salts thereof, in combination with a pharmaceutically acceptable carrier, diluent or excipient.
